Europäisches Patentamt

European Patent Office

Office européen des brevets

(19)

(11)  **EP 0 536 128 B1**

(12)  **EUROPÄISCHE PATENTSCHRIFT**

(45) Veröffentlichungstag und Bekanntmachung des Hinweises auf die Patenterteilung:
**31.07.1996  Patentblatt 1996/31**

(21) Anmeldenummer: **91900021.6**

(22) Anmeldetag: **20.11.1990**

(51) Int. Cl.$^6$: **A61L 15/00**, C08J 3/12

(86) Internationale Anmeldenummer:
**PCT/EP90/01981**

(87) Internationale Veröffentlichungsnummer:
**WO 92/00108 (09.01.1992  Gazette 1992/02)**

(54) **PULVERFÖRMIGE, VERNETZTE, WÄSSRIGE FLÜSSIGKEITEN SOWIE BLUT ABSORBIERENDE POLYMERE, VERFAHREN ZU IHRER HERSTELLUNG UND IHRE VERWENDUNG ALS ABSORPTIONSMITTEL IN HYGIENEARTIKELN**

AQUEOUS-LIQUID AND BLOOD-ABSORBING POWDERY RETICULATED POLYMERS, PROCESS FOR PRODUCING THE SAME AND THEIR USE AS ABSORBENTS IN SANITARY ARTICLES

POLYMERES PULVERULENTS RETICULES ABSORBANT DES LIQUIDES AQUEUX ET DU SANG, LEUR PROCEDE DE PRODUCTION ET LEUR UTILISATION COMME ABSORBANTS DANS DES ARTICLES HYGIENIQUES

(84) Benannte Vertragsstaaten:
**AT BE CH DE DK ES FR GB IT LI LU NL SE**

(30) Priorität: **29.06.1990 DE 4020780**

(43) Veröffentlichungstag der Anmeldung:
**14.04.1993  Patentblatt 1993/15**

(73) Patentinhaber: **Chemische Fabrik Stockhausen GmbH**
**D-47805 Krefeld (DE)**

(72) Erfinder:
• **DAHMEN, Kurt**
  **D-4050 Mönchengladbach-Rheydt (DE)**
• **MERTENS, Richard**
  **D-4150 Krefeld (DE)**

(74) Vertreter: **Klöpsch, Gerald, Dr.-Ing.**
**Patentanwalt**
**An Gross St. Martin 6**
**D-50667 Köln (DE)**

(56) Entgegenhaltungen:
**EP-A- 0 239 223          EP-A- 0 339 461**
**EP-A- 0 361 842          US-E-   32 649**

**Beschreibung**

Die Erfindung betrifft pulverförmige, vernetzte, wässrige Flüssigkeiten sowie Blut absorbierende Polymere (Superabsorber) mit verbesserten Eigenschaften hinsichtlich Quellung und Rückhaltevermögen von wäßrigen Flüssigkeiten unter Belastung, ein Verfahren zur Herstellung dieser Polymeren sowie ihre Verwendung in absorbierenden Sanitärartikeln, wie bei Babywindeln, bei der Erwachseneninkontinenz, der Damenhygiene und der Wundabdeckung.

Superabsorber sind wasserunlösliche, vernetzte Polymere, die in der Lage sind, unter Quellung und Ausbildung von Hydrogelen große Mengen an wäßrigen Flüssigkeiten und Körperflüssigkeiten, wie z.B. Urin oder Blut, aufzunehmen und die absorbierte Flüssigkeitsmenge unter einem bestimmten Druck zurückzuhalten. Durch diese charakteristischen Absorptionseigenschaften finden die Polymeren hauptsächlich Anwendung bei der Einarbeitung in Sanitärartikel, wie z.B. in Babywindeln und Damenbinden.

Bei den gegenwärtig kommerziell verfügbaren Superabsorbern handelt es sich um vernetzte Polyacrylsäuren oder vernetzte Stärke-Acrylsäure-Pfropfpolymerisate, bei denen die Carboxylgruppen teilweise mit Natronlauge oder Kalilauge neutralisiert sind.

Die Herstellung der pulverförmigen Superabsorber erfolgt prinzipiell nach zwei Methoden:

Nach der ersten Methode wird teilneutralisierte Acrylsäure in wäßriger Lösung in Gegenwart eines mehrfunktionellen Vernetzers durch radikalische Polymerisation in ein Gel überführt, das dann zerkleinert, getrocknet, gemahlen und auf die gewünschte Partikelgröße abgesiebt wird. Diese Lösungspolymerisation kann kontinuierlich oder diskontinuierlich durchgeführt werden. Die Patentliteratur lehrt ein breites Spektrum an Variationsmöglichkeiten hinsichtlich Konzentrationsverhältnissen, Temperatur, Art und Menge an Vernetzern und Initiatoren. Typische Verfahren sind z.B. beschrieben in US-PS 4,286,082, DE-PS 27 06 135 und US-PS 4,076,663.

Die zweite Methode ist das inverse Suspensions- oder Emulsionspolymerisationsverfahren. In diesem Prozeß wird eine wäßrige, teilneutralisierte Acrylsäurelösung mit Hilfe von Schutzkolloiden oder Emulgatoren in einem hydrophoben, organischen Lösungsmittel dispergiert und durch Radikalinitiatoren die Polymerisation gestartet. Nach Beendigung der Polymerisation wird das Wasser aus dem Reaktionsgemisch azeotrop entfernt und das Polymerprodukt abfiltriert und getrocknet. Die Vernetzungsreaktion kann durch Einpolymerisieren eines polyfunktionellen Vernetzers, der in der Monomerenlösung gelöst ist, und/oder durch Reaktion geeigneter Vernetzungsmittel mit funktionellen Gruppen des Polymeren während einer der Herstellungsschritte erfolgen. Das Verfahrensprinzip ist z.B. in US-PS 4,340,706, DE-PS 37 13 601 und DE-PS 28 40 010 beschrieben.

Während in der Entwicklung der Superabsorber zunächst allein das sehr hohe Quellvermögen bei Kontakt mit Flüssigkeit, auch freie Quellkapazität genannt, im Vordergrund stand, hat sich später gezeigt, daß es nicht nur auf die Menge der absorbierten Flüssigkeit ankommt, sondern auch auf die Festigkeit des gequollenen Gels. Absorptionsvermögen oder auch Quellvermögen oder freie Quellkapazität genannt einerseits und Gelfestigkeit bei einem vernetzten Polymeren andererseits stellen jedoch gegenläufige Eigenschaften dar, wie bereits aus der US-PS 3,247,171 bekannt ist, ferner aus der US-PS Re 32, 649. Das bedeutet, daß Polymere mit besonders hohem Absorptionsvermögen nur eine geringe Festigkeit des gequollenen Gels aufweisen mit der Folge, daß das Gel unter einem angewendeten Druck (z.B. Körperdruck) deformierbar ist und die weitere Flüssigkeitsverteilung und -aufnahme verhindert. Nach der US-PS Re 32,649 soll daher ein ausgewogenes Verhältnis zwischen Absorptionsvermögen (Gelvolumen) und Gelstärke angestrebt werden, damit bei der Verwendung derartiger Superabsorber in einer Windelkonstruktion Flüssigkeitsaufnahme, Flüssigkeitstransport, Trockenheit der Windel und der Haut gewährleistet sind. Dabei kommt es nicht nur darauf an, daß das Polymer Flüssigkeit unter nachfolgender Einwirkung eines Drucks zurückhalten kann, nachdem das Polymer zunächst frei quellen konnte, sondern auch darauf, Flüssigkeiten auch gegen einen gleichzeitig, d.h. während der Flüssigkeitsabsorption, ausgeübten Druck aufzunehmen, wie es unter praktischen Gegebenheiten geschieht, wenn ein Baby oder eine Person auf einem Sanitärartikel sitzt oder liegt oder wenn es, z.B. durch Beinbewegungen, zur Einwirkung von Scherkräften kommt. Diese spezifische Absorptionseigenschaft wird in der EP 0 339 461 als Aufnahme unter Druck bezeichnet.

Der zunehmenden Tendenz, aus verständlichen ästhetischen Gründen und aus Umweltaspekten (Verringerung des Deponievolumens) die Sanitärartikel immer kleiner und dünner zu gestalten, kann nur dadurch entsprochen werden, daß man den großvolumigen Fluffanteil in Windeln reduziert und gleichzeitig den Anteil an Superabsorber erhöht. Hierdurch muß der Superabsorber zusätzliche Aufgaben bezüglich Flüssigkeitsaufnahme und -transport übernehmen, die vorher der Fluff erfüllte, welche die bisher bekannten Superabsorber nicht ausreichend erfüllen können.

Aufgabe der vorliegenden Erfindung ist es daher, superabsorbierende Polymere bereitzustellen, die die charakteristische Eigenschaftskombination wie hohe Retentionskapazität, hohe Gelstärke und hohes Aufnahmevermögen unter Druck in besonderem Maße besitzen. Eine weitere Aufgabe der vorliegenden Erfindung ist die Bereitstellung eines Verfahrens für die Herstellung solcher Absorptionsmittel.

Diese Aufgabe wird gelöst durch die kennzeichnenden Merkmale des Anspruchs 1. Überraschenderweise hat sich gezeigt, daß durch die Beschichtung eines teilchenförmigen Absorberharzes mit 0,1 bis 5 Gew% Alkylencarbonaten und anschließende Erhitzung auf 150 bis 300°C eine bedeutende Verbesserung der Aufnahme unter Druck bei gleichzeitig hohen Retentionswerten und hohen Gelstärken erzielt werden kann.

Die Oberflächenbehandlung von wasserabsorbierenden Harzen ist bereits bekannt. So werden in der US-PS 4,043,952 zur Verbesserung der Dispergierbarkeit in Wasser polyvalente Metallverbindungen und in der US-PS 4,051,086 zur Verbesserung der Aufnahmegeschwindigkeit Glyoxal empfohlen. In den Patentschriften EP 0 083 022 (zur verbesserten Dispergierbarkeit in Wasser und zur Verbesserung des Absorptionsvermögens), DE-OS 33 31 644 (zur Verbesserung der Resistenz gegen Salzlösungen bei hoher Wasseraufnahmegeschwindigkeit), DE-OS 35 07 775 (ebenfalls zur Erhöhung der Salzbeständigkeit bei guter Flüssigkeitsabsorption und Gelfestigkeit), DE-OS 35 23 617 (zur Verbesserung der Fließfähigkeit und dem Verhindern des Zusammenbackens), DE-OS 36 28 482 (zur Verbesserung der Wasseraufnahme bei wiederholter Verwendung) und EP 0 349 240 (zur Erzielung eines Gleichgewichtes zwischen Absorptionsvermögen und Absorptionsgeschwindigkeit sowie Gelfestigkeit und Saugkraft) wird die Nachbehandlung von Harzen mit zwei- oder mehrfunktionelle Gruppen enthaltenden Vernetzungsmitteln beschrieben, die mit den Carboxyl- oder Carboxylatgruppen oder anderen im Polymer enthaltenen Gruppen reagieren können. Hierbei wird entweder das Pulver direkt mit den Komponenten, ggf. unter Mitverwendung geringer Mengen Wasser und Lösungsmittel, vermischt oder das Pulver in einem inerten Lösungsmittel dispergiert oder 10 bis 40 Gew% Wasser enthaltende Polymere werden in einem hydrophilen oder hydrophoben Lösungsmittel dispergiert und anschließend oder gleichzeitig mit dem Vernetzungsmittel vermischt. Als Vernetzungsmittel können Polyglydicylether, Haloepoxiverbindungen, Polyole, Polyamine oder Polyisocyanate verwendet werden. Neben diesen werden in DE-OS 33 14 019, EP 0 317 106 (jeweils zur Erreichung von hoher Absorptionsmenge und hoher Absorptionsgeschwindigkeit) und DE-OS 37 37 196 (hohes Absorptionsvermögen und hohe Aufnahmegeschwindigkeit bei gleichzeitiger großer Gelfestigkeit) weiterhin polyfunktionelle Aziridinverbindungen, Alkyl-di-(tri)-halogenide und öllösliche Polyepoxiverbindungen erwähnt. In der DE-OS 35 03 458 (zur Erzielung eines Polymeren mit gutem Wasserabsorptionsvermögen, hoher Wasserabsorptionsrate und hoher Gelfestigkeit bei nicht-klebrigem Gel) erfolgt die Aufbringung eines Vernetzungsmittels auf ein Polymerharz in Gegenwart eines inerten anorganischen Pulvermaterials wie $SiO_2$ ohne Verwendung organischer Lösungsmittel. Allen diesen Verfahren ist gemeinsam, daß anschließend eine Temperaturbehandlung der Harze erfolgt, sowie ferner, daß die zur Oberflächenbehandlung verwendeten Vernetzer wenigstens zwei funktionelle Gruppen aufweisen.

Einen Hinweis darauf, daß durch eine Oberflächenbehandlung von Absorberharzen die Aufnahme unter Druck gesteigert werden kann oder daß die Eigenschaftskombination von hoher Retentionskapazität, hoher Gelstärke und hohem Aufnahmevermögen unter Druck gleichzeitig erreicht werden kann, läßt der vorstehend beschriebene Stand der Technik nicht erkennen.

Die meisten der bisher verwendeten Vernetzungsmittel haben nachteilige toxische Eigenschaften. Sie können deshalb auf einem so sensitiven Gebiet wie dem Hygienesektor wegen des gesundheitsgefährdenden Potentials nicht verwendet werden. Neben der noch relativ harmlosen Gefahr einer Hautreizung zeigen Epoxy-, Glycidyl-, organische Halogenverbindungen und Isocyanate sensibilisierende Wirkung und vielfach ein cancerogenes und mutagenes Potential. Der Einsatz von Polyaminen verbietet sich wegen möglicher Nitrosaminbildung. Jedenfalls muß für den Einsatz in Babywindeln und anderen Hygieneartikeln eine sorgfältige Entfernung nicht reagierter Anteile der toxikologisch bedenklichen Vernetzungsmittel aus den Polymerharzen erfolgen. Das bedingt zusätzliche und aufwendige Reinigungsschritte, die die bekannten Herstellungsprozesse von der Kostenseite stark belasten und unwirtschaftlich gestalten.

Erfindungsgemäß können als Alkylencarbonate z.B. eingesetzt werden: 1,3-Dioxolan-2-on, 4-Methyl-1,3-dioxolan-2-on, 4,5-Dimethyl-1,3-dioxolan-2-on, 4,4-Dimethyl-1,3-dioxolan--2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on oder 1,3-Dioxepan-2-on. Besonders bevorzugt werden 1,3-Dioxolan-2-on und 4-Methyl-1,3-dioxolan-2-on.

Das wasserabsorbierende Harz, das zur Beschichtung verwendet werden kann, wird erhalten durch Polymerisation von 55 - 99,9 Gew% Monomeren mit Säuregruppen, wie z.B. Acrylsäure, Methacrylsäure, 2-Acrylamido-2-methylpropansulfonsäure oder Mischungen dieser Monomeren; die Säuregruppen liegen mindestens zu 25 Mol% neutralisiert vor, so z.B. als Natrium-, Kalium- oder Ammoniumsalze. Bevorzugt liegt der Neutralisationsgrad bei mindestens 50 Mol%. Besonders bevorzugt ist ein Harz, das aus vernetzter Acrylsäure oder Methacrylsäure gebildet ist, die zu 50 - 80 Mol% neutralisiert ist.

Als weitere Monomere können für die Herstellung der wasserabsorbierenden Harze 0 - 40 Gew% Acrylamid, Methacrylamid, Hydroxyethylacrylat, Dimethylaminoalkyl(meth)-acrylat, Dimethylaminopropylacrylamid oder Acrylamidopropyltrimethylammoniumchlorid verwendet werden. Höhere Anteile als 40 % dieser Monomerer verschlechtern die Quellfähigkeit der Harze.

Als Vernetzer können alle Verbindungen verwendet werden, die mindestens zwei ethylenisch ungesättigte Doppelbindungen oder eine ethylenisch ungesättigte Doppelbindung und eine gegenüber Säuregruppen reaktive funktionelle Gruppe oder mehrere gegenüber Säuregruppen reaktive funktionelle Gruppen tragen. Beispielhaft seien genannt: Acrylate und Methacrylate von Polyolen, wie Butandiol-diacrylat, Hexandiol-dimethacrylat, Polyglykol-diacrylat, Trimethylolpropantriacrylat, oder Allylacrylat, Diallylacrylamid, Triallylamin, Diallylether, Methylenbisacrylamid oder N-methylolacrylamid.

Als wasserlösliche Polymere können im wasserabsorbierenden Harz 0 - 30 Gew% teil- oder vollverseifter Polyvinylalkohol, Polyvinylpyrrolidon, Stärke oder Stärkederivate, Polyglykole oder Polyacrylsäuren enthalten sein. Das Molekulargewicht dieser Polymeren ist unkritisch, solange sie wasserlöslich sind. Bevorzugte wasserlösliche Polymere sind Stärke oder Polyvinylalkohol oder Gemische dieser Polymeren. Der bevorzugte Gehalt an solchen wasserlöslichen Polymeren im wasserabsorbierenden Harz liegt bei 1 - 5 Gew%, insbesondere wenn Stärke und/oder Polyvinylalkohol als lösliche Polymere vorhanden sind.Die wasserlöslichen Polymere können als Pfropfpolymere mit den Säuregruppen enthaltenden Polymeren vorliegen.

Bevorzugt werden neben Harzen, die durch vernetzende Polymerisation teilneutralisierter Acrylsäure erhalten worden sind, solche verwendet, die zusätzlich Anteile von pfropfpolymerisierter Stärke oder von Polyvinylalkohol enthalten.

Hinsichtlich der Teilchenform des eingesetzten Absorberharzes gibt es keine speziellen Einschränkungen. Das Polymer kann in Form von Kügelchen vorliegen, die durch inverse Suspensionspolymerisation erhalten wurden oder von unregelmäßig geformten Teilchen, die durch Trocknung und Pulverisierung der Gelmasse aus der Lösungspolymerisation stammen. Die Teilchengröße liegt normalerweise zwischen 20 und 2000 $\mu$m, bevorzugt zwischen 50 und 850 $\mu$m.

Zur Beschichtung können die wasserabsorbierenden Harze mit einer wäßrig-alkoholischen Lösung des Alkylencarbonats vermischt werden. Die Menge Alkohol wird von der Löslichkeit des Alkylencarbonats bestimmt und wird aus technischen Gründen, wie z.B. Explosionsschutz, so gering wie möglich gehalten. Geeignete Alkohole sind Methanol, Ethanol, Butanol oder Butylglykol sowie Gemische dieser Alkohole. Bevorzugtes Lösungsmittel ist Wasser, das in einer Menge von 0,3 - 5,0 Gew.%, bezogen auf Harz, vewendet wird. Es besteht auch die Mögichkeit, das Alkylencarbonat aus einer Pulvermischung, z.B. mit einem anorganischen Trägermaterial wie $SiO_2$, aufzubringen.

Um die gewünschten Eigenschaften zu erzielen, ist eine gleichmäßige Verteilung des Alkylencarbonats auf dem Harzpulver erforderlich. Dazu führt man die Vermischung in geeigneten Mischern durch, wie z.B. Wirbelbettmischer, Schaufelmischer, Walzenmischer oder Doppelschneckenmischer.

Es besteht auch die Möglichkeit, die Beschichtung des Absorberharzes während eines der Verfahrensschritte bei der Herstellung des Polymerharzes vorzunehmen. Hierzu ist besonders der Prozeß der inversen Suspensionspolymerisation geeignet.

Die sich an die Beschichtung anschließende thermische Behandlung wird bei 150 - 300°C durchgeführt, bei Verwendung der bevorzugten Alkylencarbonate bei 180 - 250°C. Sie ist abhängig von der Verweilzeit und der Art des Alkylencarbonats. Bei 150°C muß die thermische Behandlung über mehrere Stunden durchgeführt werden, während bei 250°C wenige Minuten, z.B. 0,5 bis 5 Minuten ausreichen, um die gewünschten Eigenschaften zu erzielen. Die thermische Behandlung-kann in üblichen Trocknern oder Öfen durchgeführt werden; beispielhaft seien Drehrohröfen, Wirbelbetttrockner, Tellertrockner oder Infrarottrockner genannt.

Die Polymeren gemäß der Erfindung können in großtechnischer Weise nach kontinuierlichen und diskontinuierlichen Verfahren hergestellt werden. Die erfindungsgemäßen Mittel können für weite Anwendungsgebiete eingesetzt werden. Wenn sie z.B. als Absorbierungsmittel in Damenbinden und Windeln oder zur Wundabdeckung verwendet werden, dann besitzen sie die Eigenschaft, daß sie große Mengen an Menstruationsblut, Urin oder anderen Körperflüssigkeiten schnell absorbieren. Die Absorptionsfähigkeit und -geschwindigkeit unter gleichzeitig einwirkender Druckbelastung ist viel höher als bei bekannten Produkten. Da die erfindungsgemäßen Mittel die absorbierten Flüssigkeiten auch unter Druck zurückhalten, sind die Mittel besonders anwendungsfreundlich. Sie sind bevorzugt geeignet, in höheren Konzentrationen, bezogen auf hydrophiles Fasermaterial wie z.B. Fluff, eingesetzt zu werden, als dies bisher möglich war, und zeigen ausgezeichnete Absorptionseigenschaften in Konstruktionen, die 98 bis 20 Gew% hydrophile Fasern und 2 bis 80 Gew% des Absorberharzes enthalten.

Die erfindungsgemäß beschichteten Polymeren werden in Absorberartikeln für die verschiedensten Anwendungszwecke eingesetzt, z.B. durch Mischen mit Papier oder Fluff oder synthetischen Fasern oder Verteilen des Mittels zwischen Substraten aus Papier, Fluff oder nichtgewebten Textilien oder durch Verformung in Trägermaterialien zu einer Bahn.

Die für die Beschichtung von Polymerharzen erfindungsgemäß verwendeten Alkylencarbonate besitzen keine toxikologisch bedenklichen Eigenschaften.

Die durch Beschichtung mit den erfindungsgemäßen Alkylencarbonaten und nachfolgende Erhitzung erhaltenen Superabsorber weisen überraschenderweise eine bedeutende Verbesserung der Aufnahme von Flüssigkeit unter Druck hinsichtlich Geschwindigkeit und Gesamtkapazität bei gleichzeitig hoher Gelstärke und hohen Retentionen auf, wobei insbesondere eine sehr hohe Anfangsgeschwindigkeit der Flüssigkeitsaufnahme unter Druck erreicht wird, so daß 80 % der Gesamtkapazität bereits nach 15 Minuten erreicht werden. Die Aufnahme unter Druck (AUL) liegt über 25 g/g bei einer Belastung von 20 g/cm$^2$, bevorzugt über 27 g/g bei Retentionswerten (TB) von mindestens 28 g/g, vorzugsweise über 30 g/g, wenn die Absorption mit 0,9%iger Natriumchloridlösung gemessen wird. Die Summe aus Retention und Aufnahme unter Druck ist größer als 53 g/g, vorzugsweise größer als 60 g/g. Die Gelfestigkeit der erfindungsgemäßen Produkte beträgt bei einem Gelvolumen von 28 g/g mindestens 2000 N/m$^2$.

Testmethoden:

Zur Charakterisierung der wasserabsorbierenden Harze wurden Retention (TB), Aufnahme unter Druck (AUL) und Schermodul gemessen.

Die Retention wird nach der Teebeutelmethode bestimmt und als Mittelwert aus drei Messungen angegeben. Ca. 200 mg Harz werden in einen Teebeutel eingeschweißt und für 20 Minuten in 0,9%ige NaCl-Lösung getaucht. Anschließend wird der Teebeutel in einer Schleuder (23 cm Durchmesser, 1.400 Upm) 5 Minuten geschleudert und gewogen. Einen Teebeutel ohne wasserabsorbierendes Harz läßt man als Blindwert mitlaufen:

$$\text{Retention} = \frac{\text{Auswaage - Blindwert}}{\text{Einwaage}} \text{ (g/g)}$$

Die Aufnahme unter Druck (Druckbelastung 20 g/cm$^2$) wird nach der in der EP 0 339 461, Seite 7, beschriebenen Methode bestimmt: In einen Zylinder mit Siebboden gibt man die Einwaage an Superabsorber und belastet das Pulver mit einem Stempel, der einen Druck von 20 g/cm$^2$ ausübt. Der Zylinder wird anschließend auf einen Demand-Absorbency-Tester (DAT) gestellt, wo man den Superabsorber eine Stunde lang 0,9%ige NaCl-Lösung saugen läßt.

Der Schermodul wird mit einem Carri-Med-Stress-Rheometer mit einer Platte-Platte-Konfiguration gemessen. Zur Bestimmung des Schermoduls läßt man 1 g wasserabsorbierendes Harz in 28 g 0,9%iger NaCl-Lösung für 1 Std. quellen und mißt anschließend an diesem gequollenen Gel den Schermodul in Abhängigkeit von der Frequenz (0,1 - 10 Hz). Der Wert bei 10 Hz wird als Speichermodul G' angegeben.

Die wasserabsorbierenden Harzpulver, die in den nachfolgenden Beispielen zur Beschichtung mit einem Alkylencarbonat verwendet werden, wurden nach den bekannten Verfahren der Lösungs- oder Suspensionspolymerisation hergestellt. Alle %-Angaben beziehen sich auf Pulver.

## BEISPIEL 1

Eine pulverförmige, mit Trimethylolpropantriacrylat vernetzte Polyacrylsäure, die zu 70 Mol% als Natriumsalz neutralisiert vorlag, wurde auf 50 - 850 µm abgesiebt (Pulver A). 100 g Pulver A wurden unter kräftigem Rühren mit einer Lösung aus je 2,5 g 1,3-Dioxolan-2-on, Wasser und Ethanol vermischt und anschließend für 1 Std. in einem Ofen, der auf 180°C temperiert war, erhitzt.

Zum Vergleich wurden 100 g Pulver A mit einer Mischung aus 2,5 g Wasser und 2,5 g Ethanol vermischt und ebenfalls 1 Std. bei 180° erhitzt.

Nach dem Abkühlen wurden die Pulver nochmals auf 50 - 850 µm gesiebt, und es wurden die Retention (TB), die Aufnahme unter Druckbelastung (AUL) sowie der Speichermodul G' bestimmt:

| | TB g/g | AUL g/g | Summe TB+AUL | G' N/m$^2$ |
|---|---|---|---|---|
| Pulver A | 45 | 6 | 51 | 1200 |
| Beispiel 1 | 41 | 33 | 74 | 2600 |
| Vergleichsbeispiel ohne 1,3-Dioxolan-2-on | 45 | 6 | 51 | 1200 |

## BEISPIELE 2 - 4

Drei pulverförmige, unterschiedlich stark vernetzte Polyacrylsäuren, die zu 70 Mol% als Natriumsalz neutralisiert vorlagen (Pulver B, C, D), wurden entsprechend Beispiel 1 mit 1,3-Dioxolan-2-on vermischt und für 1 Std. bei 180° C im Ofen erhitzt:

| | 1,3-Dioxolan-2-on % / H$_2$O% / Ethanol % | | | T B g/g | AUL g/g |
|---|---|---|---|---|---|
| Pulver B | --- | | | 39 | 11 |
| Beispiel 2 | 1,5 | 2,0 | 2,0 | 36 | 30 |
| Pulver C | --- | | | 36 | 13 |
| Beispiel 3 | 1,0 | 2,0 | 2,0 | 34 | 31 |
| Pulver D | --- | | | 31 | 17 |
| Beispiel 4 | 0,2 | 1,0 | 2,0 | 30 | 30 |
| Fortsetzung: | | | | Summe TB+AUL | G' N/m$^2$ |
| Pulver B | | | | 50 | 1800 |
| Beispiel 2 | | | | 66 | 3000 |
| Pulver C | | | | 49 | 2300 |
| Beispiel 3 | | | | 65 | 3200 |
| Pulver D | | | | 48 | 4000 |
| Beispiel 4 | | | | 60 | 4200 |

## BEISPIELE 5 - 8

100 g Pulver B wurden mit verschiedenen Carbonaten, gelöst in einer Mischung aus Wasser und Ethanol, vermischt und in einem Ofen bei 215°C erhitzt.

| | Carbonat | Menge Carbonat | Zeit Min. | TB g/g | AUL g/g |
|---|---|---|---|---|---|
| Pulver B | | - | - | 39 | 11 |
| Beispiel 5 | 4-Methyl-1,3-dioxolan-2-on | 2 g | 20 | 37 | 31 |
| Beispiel 6 | 1,3-Dioxan-2-on | 2 g | 15 | 37 | 30 |
| Beispiel 7 | 4-Methyl-1,3-dioxan-2-on | 2 g | 30 | 36 | 33 |
| Beispiel 8 | 4-Ethyl-1,3-dioxolan-2-on | 2 g | 20 | 37 | 30 |
| Fortsetzung: | | | | Summe TB+AUL | G' N/m$^2$ |
| Pulver B | | | | 50 | 1800 |
| Beispiel 5 | | | | 68 | 2500 |
| Beispiel 6 | | | | 67 | 2600 |
| Beispiel 7 | | | | 69 | 2600 |
| Beispiel 8 | | | | 67 | 2400 |

## BEISPIELE 9 - 13

Verschiedene Mengen 1,3-Dioxolan-2-on bzw. Wasser wurden mit 100 g Pulver A vermischt und 1 Std. bei 180°C in einem Ofen erhitzt.

| | 1,3-Dioxolan-2-on % / $H_2O$ % | | TB g/g | AUL g/g | Summe TB+AUL | G' $N/m^2$ |
|---|---|---|---|---|---|---|
| Pulver A | - | - | 45 | 6 | 51 | 1200 |
| Beispiel 9 | 0,5 | 0,5 | 43 | 28 | 71 | 2350 |
| Beispiel 10 | 1,0 | 1,0 | 41 | 32 | 73 | 2450 |
| Beispiel 11 | 1,5 | 1,5 | 40 | 34 | 74 | 2500 |
| Beispiel 12 | 2,0 | 2,0 | 37 | 34 | 71 | 2700 |
| Beispiel 13 | 3,5 | 3,5 | 32 | 32 | 67 | 2800 |

## BEISPIELE 14 - 19

100 g Pulver B wurden mit einer wäßrigen Lösung 1,3-Dioxolan-2-on vermischt und mit der erwärmten Luft eines Heißluftgebläses auf verschiedene Temperaturen erwärmt. Die Temperatur des Luftstroms wurde vor Kontakt mit dem Pulver gemessen.

| | EC* | $H_2O$ | T ° C | Zeit Min. | TB g/g | AUL g/g | Summe TB + AUL |
|---|---|---|---|---|---|---|---|
| | % | | | | | | |
| Pulver B | - | - | - | - | 39 | 11 | 50 |
| Beispiel 14 | 2,5 | 2,0 | 215 | 5 | 38 | 28 | 66 |
| Beispiel 15 | 2,5 | 2,0 | 215 | 10 | 36 | 29 | 65 |
| Beispiel 16 | 2,5 | 2,5 | 215 | 20 | 34 | 28 | 62 |
| Beispiel 17 | 1,0 | 1,5 | 250 | 2 | 38 | 29 | 67 |
| Beispiel 18 | 1,0 | 1,0 | 250 | 5 | 36 | 29 | 65 |
| Beispiel 19 | 1,0 | 1,0 | 250 | 10 | 34 | 30 | 64 |

\* EC = 1,3-Dioxolan-2-on

## BEISPIELE 20 - 23

Pulverförmige, vernetzte, Stärke bzw. Polyvinylalkohol enthaltende Polyacrylsäuren, die zu 70 Mol% als Natriumsalz neutralisiert vorlagen (Pulver E, F, G, H), wurden mit wäßrig-alkoholischen Lösungen von 1,3-Dioxolan-2-on vermischt und 2 Std. bei 170°C in einem Ofen erwärmt:

| | PVA[1] | Stärke | EC | $H_2O$ | EtOH[2] | TB g/g | AUL g/g |
|---|---|---|---|---|---|---|---|
| | % | | % | | | | |
| Pulver E | 3,5 | - | | --- | | 45 | 7 |
| Beispiel 20 | 3,5 | - | 2 | 1 | 0 | 37 | 30 |
| Pulver F | 4,5 | - | | --- | | 45 | 6 |
| Beispiel 21 | 4,5 | - | 1 | 1 | 1 | 41 | 28 |
| Pulver G | - | 3,5 | | --- | | 41 | 7 |
| Beispiel 22 | - | 3,5 | 1,5 | 1 | 1 | 35 | 29 |
| Pulver H | - | 6,0 | | --- | | 40 | 7 |
| Beispiel 23 | - | 6,0 | 1 | 1 | 1 | 34 | 29 |

| Fortsetzung: | Summe TB + AUL | | $G'\ N/m^2$ | |
|---|---|---|---|---|
| Pulver E | 52 | | 1300 | |
| Beispiel 20 | 67 | | 2400 | |
| Pulver F | 51 | | 1200 | |
| Beispiel 21 | 69 | | 2100 | |
| Pulver G | 48 | | 1600 | |
| Beispiel 22 | 64 | | 2200 | |
| Pulver H | 47 | | 1600 | |
| Beispiel 23 | 63 | | 2300 | |

[1] PVA = Polyvinylalkohol
[2] EtOH = Ethanol

## BEISPIELE 24 - 26

Vernetzte, pulverförmige Copolymere aus Acrylsäure/2-Acrylamido-2-methylpropansulfonsäure (Pulver K), Acrylsäure/-Acrylamid (Pulver L) und Acrylsäure/Dimethylaminopropylacrylamid (Pulver M) wurden mit wäßrig-äthanolischen Lösungen von 1,3-Dioxolan-2-on vermischt und 15 Minuten bei 215°C in einem Ofen erhitzt.

| | AcS / Comonomer[1] Gew% | EC | $H_2O$ | EtOH[1] | TB g/g |
|---|---|---|---|---|---|
| | | | % | | |
| Pulver K | 65 / 35 AMPS | | --- | | 35 |
| Beispiel 24 | " | 0,3 | 1,0 | 2,0 | 34 |
| Pulver L | 80 / 20 AcA | | --- | | 37 |
| Beispiel 25 | " | 0,5 | 1,0 | 1,0 | 34 |
| Pulver M | 90 / 10 DIMAPA | | --- | | 39 |
| Beispiel 26 | " | 0,5 | 1,0 | 1,0 | 36 |
| **Fortsetzung:** | | **AUL g/g** | **Summe TB+AUL** | | **G' N/m$^2$** |
| Pulver K | | 7 | 42 | | 2700 |
| Beispiel 24 | | 29 | 63 | | 3450 |
| Pulver L | | 6 | 43 | | 1900 |
| Beispiel 25 | | 28 | 62 | | 3000 |
| Pulver M | | 6 | 45 | | 1600 |
| Beispiel 26 | | 28 | 64 | | 2350 |

[1] AMPS = 2-Acrylamido-2-methylpropansulfonsäure AcA = Acrylamid DIMAPA = Dimethylaminopropylacrylamid EC = 1,3-Dioxolan-2-on EtOH = Ethanol

## BEISPIELE 27 - 30

Die Verwendung der erfindungsgemäßen, wasserabsorbierenden Harze wurde in schichtartig aufgebauten Konstruktionen aus Fluff und wasserabsorbierendem Harz geprüft. Runde Konstruktionen (Durchmesser 5,8 cm) aus 3 Fluff-Schichten und 2 Schichten wasserabsorbierendem Harz wurden in einen Büchnertrichter gelegt und mit 20g/cm$^2$ belastet. Der Büchnertrichter ist über einen Schlauch mit einem Reservoir von 0,9%iger NaCl-Lösung verbunden. Man läßt die Konstruktion 15 Minuten bzw. 1 Std. lang saugen, schweißt sie anschließend in einen großen Teebeutel ein und schleudert in einer Trommel mit 23 cm Durchmesser für 5 Minuten bei 1400 Upm. Die Aufnahme des Harzes wird wie folgt berechnet:

$$\text{Aufnahme} = \frac{\text{g (Teebeutel mit Fluff/Harz) - g (Teebeutel mit Fluff/ ohne Harz)}}{\text{Einwaage Harz}}$$

| | Wasserabsorbierendes Harz aus Beispiel | % Anteil Harz in der Konstruktion | Aufnahme g/g | |
|---|---|---|---|---|
| | | | 15 Min | 60 Min |
| Beispiel 27 | 1 | 40 | 26 | 32 |
| Beispiel 28 | 11 | 40 | 27 | 34 |
| Beispiel 29 | 13 | 40 | 25 | 30 |
| Beispiel 30 | 20 | 40 | 25 | 28 |
| Vergleich : | Pulver B | 40 | 10 | 16 |
| | Pulver D | 40 | 16 | 20 |

**Patentansprüche**

1. Pulverförmiges, wasserunlösliches, vernetztes, wäßrige oder seröse Flüssigkeiten sowie Blut absorbierendes Harz, gebildet aus

   a) 55 - 99,9 Gew% polymerisierten ungesättigten, polymerisierbaren, säuregruppenenthaltenden Monomeren, die mindestens zu 25 Mol% neutralisiert sind,

   b) 0 - 40 Gew% polymerisierten ungesättigten, mit a) copolymerisierbaren Monomeren,

   c) 0,1 - 5,0 Gew% eines Vernetzungsmittels,

   d) 0 - 30 Gew% eines wasserlöslichen Polymeren,

   wobei die Gewichtsmengen a) bis d) auf wasserfreies Polymer bezogen sind, dadurch gekennzeichnet, daß das Harzpulver mit 0,1 bis 5 Gew% eines Alkylencarbonats, bezogen auf Harzpulver, beschichtet und auf eine Temperatur von 150 bis 300°C erhitzt worden ist.

2. Absorbierendes Harz nach Anspruch 1, dadurch gekennzeichnet, daß es

   a) eine Retention von mindestens 28 g an 0,9%iger Natriumchloridlösung pro g Harz,

   b) eine Aufnahme von mindestens 25 g an 0,9%iger Natriumchloridlösung pro g Harz unter einem Druck von 20 g/cm$^2$,

   c) eine Gelstärke, gemessen als Schermodul, von mindestens 2000 N/m$^2$ bei einem Gelvolumen von 28 g 0,95iger Natriumchloridlösung pro g Harz aufweist.

3. Absorbierendes Harz nach einem der Ansprüche 1 bis 2, dadurch gekennzeichnet, daß es aus Acrylsäure, Methacrylsäure und/oder 2-Acrylamido-2-methylpropansulfonsäure als säuregruppenhaltigen Monomeren gebildet ist.

4. Absorbierendes Harz nach einem der Ansprüche 1 bis 3, dadurch gekennzeichnet, daß die Säuregruppen enthaltenen Monomeren zu mindestens 50 Mol% neutralisiert sind.

5. Wasserabsorbierendes Harz nach einem der Ansprüche 1 bis 4, dadurch gekennzeichnet, daß es aus Acrylsäure als einzigem säuregruppenenthaltenden Monomer, die zu 50 - 80 Mol% neutralisiert ist, gebildet ist.

6. Wasserabsorbierendes Harz nach einem der Ansprüche 1 bis 5, dadurch gekennzeichnet, daß wasserlösliche Polymere in Konzentrationen von 1 - 5 Gew% eingesetzt sind.

7. Wasserabsorbierendes Harz nach einem der Ansprüche 1 bis 6, dadurch gekennzeichnet, daß als wasserlösliche Polymere Stärke und/oder Polyvinylalkohol eingesetzt sind.

8. Absorbierendes Harz nach einem der Ansprüche 1 bis 7, dadurch gekennzeichnet, daß 0,2 - 3,5 Gew% Alkylencarbonate verwendet sind.

9. Absorbierendes Harz nach einem der Ansprüche 1 bis 8, dadurch gekennzeichnet, daß als Alkylencarbonate 1,3-Dioxolan-2-on und/oder 4-Methyl-1,3-Dioxolan-2-on verwendet sind.

10. Verfahren zur Herstellung eines pulverförmigen, wasserunlöslichen, vernetzten, wäßrige und seröse Flüssigkeiten sowie Blut absorbierenden Harzes, das aus

    a) 55 - 99,9 Gew% polymerisierten ungesättigten, polymerisierbaren, säuregruppenenthaltenden Monomeren, die mindestens zu 25 Mol% neutralisiert sind,

    b) 0 - 40 Gew% polymerisierten ungesättigten, mit a) copolymerisierbaren Monomeren,

    c) 0,1 - 5,0 Gew% eines Vernetzungsmittels,

d) 0 - 30 Gew% eines wasserlöslichen Polymeren,

wobei die Gewichtsmengen a) bis d) auf wasserfreies Polymer bezogen sind, besteht, <u>dadurch gekennzeichnet,</u> daß das Harzpulver mit 0,1 - 5,0 Gew% eines Alkylencarbonates, bezogen auf Harzpulver, beschichtet und auf eine Temperatur von 150 bis 300°C erhitzt wird.

11. Verfahren nach Anspruch 10, <u>dadurch gekennzeichnet,</u> daß die Beschichtung des Harzpulvers mit einer Lösung des Alkylencarbonats in Wasser oder einem Alkohol oder einem Wasser/Alkohol-Gemisch vorgenommen wird.

12. Verfahren nach Anspruch 11, <u>dadurch gekennzeichnet,</u> daß man mit einer Lösung beschichtet, die das Alkylencarbonat in einer Konzentration von 0,2 - 3,5 Gew%, bezogen auf Harz, enthält.

13. Verwendung von absorbierenden Harzen nach den Ansprüchen 1 bis 9 in Körperflüssigkeiten absorbierenden Sanitärartikeln wie Windeln, Inkontinenzhilfen, Damenbinden und Wundabdeckungen.

14. Verwendung von absorbierenden Harzen nach Ansprüchen 1 bis 9 in Absorberkonstruktionen, die zu 98 bis 20 Gew% aus hydrophilen Fasern und zu 2 bis 80 Gew% aus Absorberharz bestehen.

## Claims

1. A powdery, water-insoluble, cross-linked resin absorbing aqueous or serous liquids as well as blood made of

   a) 55 to 99.9%-wt. polymerized unsaturated, polymerizable acid-groups-containing monomers which are neutralized by at least 25 mol-%,

   b) 0 to 40%-wt. polymerized unsaturated monomers copolymerizable with a),

   c) 0.1 to 5.0%-wt. of a cross-linking agent,

   d) 0 to 30%-wt. of a water-soluble polymer,

   the weight amounts a) to d) relating to anhydrous polymer, characterized in that the resin powder has been coated with 0.1 to 5%-wt. of an alkylene carbonate, relative to resin powder, and heated to a temperature of 150 to 300°C.

2. The absorbing resin according to claim 1 exhibiting

   a) a retention of at least 28 g with 0.9%-sodium chloride solution per g of resin,

   b) an absorption of at least 25 g with 0.9%-sodium chloride solution per g of resin under a load of 20 g/cm$^2$,

   c) a gel strength, measured as shear modulus, of at least 2,000 N/m$^2$ with a gel volume of 28 g 0.95%-sodium chloride solution per g of resin.

3. The absorbing resin according to any one of claims 1 to 2, characterized in that it is formed of acrylic acid, methacrylic acid, and/or 2-acrylamido-2-methylpropane sulfonic acid as acid-groups-containing monomer.

4. The absorbing resin according to any one of claims 1 to 3 wherein the acid-groups-containing monomers are neutralized by at least 50 mol-%.

5. The water-absorbing resin according to any one of claims 1 to 4, characterized in that it is formed of acrylic acid neutralized by 50 to 80 mol-% as the only acid-groups-containing monomer.

6. The water-absorbing resin according to any one of claims 1 to 5 wherein water-soluble polymers are used in concentrations of 1 to 5%-wt.

7. The water-absorbing resin according to any one of claims 1 to 6 wherein starch and/or polyvinyl alcohol are used as water-soluble polymers.

8. The absorbing resin according to any one of claims 1 to 7 wherein 0.2 to 3.5%-wt. alkylene carbonates are used.

9. The absorbing resin according to any one of claims 1 to 8 wherein 1,3-dioxolan-2-one and/or 4-methyl-1,3-dioxolan-2-one are used as alkylene carbonates.

10. A process for the manufacture of a powdery, waterinsoluble, cross-linked resin absorbing aqueous and serous liquids as well as blood and consisting of

> a) 55 to 99.9%-wt. polymerized unsaturated, polymerizable acid-groups-containing monomers which are neutralized by at least 25 mol-%,

> b) 0 to 40%-wt. polymerized unsaturated monomers which are copolymerizable with a),

> c) 0.1 to 5.0%-wt. of a cross-linking agent,

> d) 0 to 30%-wt. of a water-soluble polymer,

the percentages by weight of a) to d) relating to anhydrous polymer, in which process the resin powder is coated with 0.1 to 5%-wt. of an alkylene carbonate, relative to resin powder, and heated to a temperature of 150 to 300°C.

11. The process according to claim 10 wherein the coating of the resin powder is carried out with a solution of the alkylene carbonate in water or in an alcohol or water/alcohol mixture.

12. The process according to claim 11 wherein coating is effected with a solution comprising the alkylene carbonate in a concentration of 0.2 to 3.5%-wt., relative to resin.

13. The use of absorbing resins as defined in claims 1 to 9 in sanitary articles which absorb body liquids, such as diapers, incontinence articles, sanitary napkins, and wound dressings.

14. The use of absorbing resins according to claims 1 to 9 in absorbent constructions consisting of 98 to 20%-wt. of hydrophilic fibers and 2 to 80%-wt. of absorbing resin.

## Revendications

1. Résine pulvérulente, insoluble dans l'eau, réticulée, absorbant les liquides aqueux ou séreux ainsi que le sang, formée de

> a) 55 à 99,9% en poids de monomères contenant des radicaux acide, polymérisables, insaturés, polymérisés, neutralisés à raison d'au moins jusqu'à 25% molaires,
> b) 0 à 40% en poids de monomères copolymérisables avec a) polymérisés, insaturés,
> c) 0,1 à 5,0% en poids d'un agent de réticulation,
> d) 0 à 30% en poids d'un polymère soluble dans l'eau,

où les proportions pondérales a) à d) se rapportent au polymère dépourvu d'eau, caractérisée en ce que la poudre de résine a été enrobée de 0,1 à 5% en poids d'un carbonate d'alkylène, par rapport à la poudre de résine, et chauffée jusqu'à une température de 150 à 300°C.

2. Résine absorbante selon la revendication 1, caractérisée en ce qu'elle présente

> a) une rétention d'au moins 28 g de solution à 0,9% de chlorure de sodium par gramme de résine,
> b) une absorption d'au moins 25 g de solution à 0,9% de chlorure de sodium par gramme de résine, sous une pression de 20 g/cm$^2$,
> c) une solidité de gel, mesurée sous forme de module de cisaillement, d'au moins 2000 N/m$^2$ pour un volume de gel de 28 g de solution à 0,95% de chlorure de sodium par gramme de résine.

3. Résine absorbante selon l'une quelconque des revendications 1 et 2, caractérisée en ce qu'elle est formée d'acide acrylique, d'acide méthacrylique et/ou d'acide 2-acrylamido-2-méthylpropanesulfonique à titre de monomères contenant des radicaux acide.

4. Résine absorbante selon l'une quelconque des revendications 1 à 3, caractérisée en ce que les monomères contenant des radicaux acide sont neutralisés à raison de jusqu'à au moins 50% molaires.

**5.** Résine absorbant l'eau selon l'une quelconque des revendications 1 à 4, caractérisée en ce qu'elle est formée d'acide acrylique à titre de seul monomère contenant des radicaux acide, qui est neutralisé à raison de jusqu'à 50 à 80% molaires.

**6.** Résine absorbant l'eau selon l'une quelconque des revendications 1 à 5, caractérisée en ce que l'on met en oeuvre des polymères solubles dans l'eau en concentrations de 1 à 5% en poids.

**7.** Résine absorbant l'eau selon l'une quelconque des revendications 1 à 6, caractérisée en ce que l'on met en oeuvre, à titre de polymères solubles dans l'eau, de l'amidon et/ou du poly(alcool vinylique).

**8.** Résine absorbante selon l'une quelconque des revendications 1 à 7, caractérisé en ce que l'on utilise de 0,2 à 3,5% en poids de carbonates d'alkylène.

**9.** Résine absorbante selon l'une quelconque des revendications 1 à 8, caractérisée en ce que l'on utilise, à titre de carbonates d'alkylène, la 1,3-dixolanne-2-one et/ou la 4-méthyl-1,3-dioxolanne-2-one.

**10.** Procédé de préparation d'une résine pulvérulente, insoluble dans l'eau, réticulée, absorbant les liquides aqueux ou séreux ainsi que le sang, formée de

    a) 55 à 99,9% en poids de monomères contenant des radicaux acide, polymérisables, insaturés, polymérisés, neutralisés à raison d'au moins jusqu'à 25% molaires,
    b) 0 à 40% en poids de monomères copolymérisables avec a) polymérisés, insaturés,
    c) 0,1 à 5,0% en poids d'un agent de réticulation,
    d) 0 à 30% en poids d'un polymère soluble dans l'eau,

où les proportions pondérales a) à d) se rapportent au polymère dépourvu d'eau, caractérisé en ce que l'on enrobe la poudre de résine de 0,1 à 5,0% en poids d'un carbonate d'alkylène, par rapport à la poudre de résine et on la chauffe jusqu'à une température de 150 à 300°C.

**11.** Procédé selon la revendication 10, caractérisé en ce que l'on entreprend l'enrobage de la poudre de résine par une solution du carbonate d'alkylène dans l'eau ou un alcool ou un mélange d'eau et d'alcool.

**12.** Procédé selon la revendication 11, caractérisé en ce que l'on procède à l'enrobage avec une solution qui contient le carbonate d'alkylène en une proportion de 0,2 à 3,5% en poids, par rapport à la résine.

**13.** Utilisation de résines absorbantes selon l'une quelconque des revendications 1 à 9, dans des articles sanitaires absorbant les liquides corporels, comme des couches, des bandages pour personnes incontinentes, des bandes cataméniales et des pansements pour plaies ou lésions.

**14.** Utilisation de résines absorbantes selon l'une quelconque des revendications 1 à 9 dans des ensembles absorbants qui se composent pour 98 à 20% en poids de fibres hydrophiles et 2 à 80% en poids de résine absorbante.